# EUROPEAN PATENT SPECIFICATION

(11) **EP 2 261 271 B1**
(45) Date of publication and mention of the grant of the patent: **30.04.2014**
(21) Application number: 10181793.0
(22) Date of filing: 17.09.2003
(51) Int. Cl.: C08F 291/00, C08F 271/00, C08F 285/00, C08F 265/00, C08F 267/00, B01D 53/00, B01J 20/00, A61M 1/36, A61K 39/00

(54) **SEPARATION MATERIAL**
TRENNUNGSMATERIAL
MATIÈRE DE SÉPARATION

(30) Priority: 17.09.2003 EP 03020986
(43) Date of publication of application: 15.12.2010
(62) Divisional of application: 03020986.0
(73) Proprietor: Gambro Lundia AB, 220 10 Lund (SE)
(72) Inventor: Storr, Markus, 71749 Filderstadt (DE); Freudemann, Wolfgang, 72379 Hechingen (DE); Müller, Egbert, 64291 Darmstadt (DE)
(74) Representative: Hornung, Veronika Margot

(56) References cited:
- EP-A- 0 052 156
- EP-A- 0 109 531
- EP-A- 0 278 100
- WO-A-01/19878
- WO-A-01/23413
- WO-A-01/92359
- WO-A-91/13098
- US-A- 4 097 420
- US-A- 5 215 692
- US-A- 5 344 560
- US-A- 5 476 509
- US-A- 5 556 708
- US-A- 5 967 714
- US-A- 6 096 800

## Description

The present invention relates to a method for the production of a separating material,

By separating material it is meant a material that is useful as an adsorption material and/or as a dialysis material and/or as a filtration material for the separation of substances, having specific chemical or physical properties, or substances, being recognized by specific recognition compounds, from a fluid, especially a liquid. Separating materials in the sense of the present invention are particularly useful in the separation or depletion, respectively, of undesired substances from liquids, e. g. the adsorptive separation of toxins from blood plasma.

Separating materials for adsorptive separation applications generally comprise a solid phase substrate material or matrix material, respectively, which carries on its surface active sites for the more or less specific adsorption of compounds having particular properties, e. g. positive or negative charges, specific chemical structures or functional groups. The solid phase substrate material may often be a porous or non-porous polymer having functional surface groups or chains of a graft copolymer being functionalized and being formed by graft polymerization of monomers onto the surface of the polymeric matrix material.

The US-A-5,556,708 describes a method for the production of an adsorption material by graft polymerization of a nitrogen-containing polymer with an ethylenically unsaturated monomer in an aqueous environment in the presence of two reactants, said two reactants consisting of carbon tetrachloride and a reducing agent, selected from sodium dithionite, rongalite, hydrazine, and ascorbic acid. According to the description and the examples of US-A-5,556,708, sodium dithionite seems to be the only one reducing agent that had been tested. Also, even though it is claimed that the nitrogen-containing polymer may be selected from polyamides, polysulfonamides, polyurethanes, and polymers having primary and secondary amine groups in a side chain, only a polyamide membrane, particularly a nylon 6,6 membrane, had been tested in the examples as the nitrogen-containing polymer. US-A-5,556,708 leaves unclear whether and how the method may work with a nitrogen-containing polymer having primary and secondary amine groups. It is known that amides form radicals with the reducing agents used according to US-A-5,556,708, but there is no mechanistical explanation how the graft polymerization should work with primary and secondary amines.

One major disadvantage of the method of US-A-5,556,708 is the prescribed use of an organic reactant, especially of carbon tetrachloride in the graft polymerization process. Even if the produced material is thoroughly cleaned after the production process, there will still be amounts of carbon tetrachloride remaining in the porous polymeric structure. The toxicity of carbon tetrachloride thus makes the produced adsorption material inappropriate for medical applications, as e. g. the adsorption of toxins from blood or in hemodialysis. On the other hand, the more or less complete removal of carbon tetrachloride from the adsorption material of US-A-5,556,708 by exhaustive rinsing or washing of the material would cause enormous costs and would make the material commercially unattractive. WO 01/92359 A1 is directed to a method for coating the surfaces of substrates with polymer materials. Active groups on the substrate are made to react with an initiator containing a leaving group, followed by graft polymerisation with at least one olefinically unsaturated monomer once the leaving group has been split. US 5,215,692 discloses a process comprising surface grafting of microporous polymers of nitrogen-containing polymers with ethylenically unsaturated monomers, wherein the process comprises removing hydrogen atoms on the nitrogen atoms of the polymer by halogen atoms using inorganic N-halogen derivatives as halogenizing agent, removing a part of the halogen by reducing agents in the presence of ethylenically unsaturated monomers during a radical grafting of the same on the nitrogen atoms and removing remaining halogen atoms by reducing agents in the absence of monomers. US 5,967,714 describes imparting antimicrobial activity to the surface of an apparatus or article by a process comprising copolymerizing tert-butylaminoethyl methacrylate with at least one other aliphatically unsaturated monomer in the presence of said apparatus or article by which adhesion of the copolymer to said surface is achieved.

Summarizing, disadvantages of prior art separating materials include the following: the substrate materials are not biocompatible or blood compatible, thus the materials are not useful for medical applications; the reactions to produce such separating materials require organic solvents which are toxic or biohazardous, thus the materials are not useful for medical applications; the reaction conditions to produce such separating materials are often harsh in a way that the preparation methods are restricted to reactants which withstand such conditions; and the reactions to produce such separating materials, if UV activation is used, do not provide for a uniform functionalization over the entire surface of a porous polymeric matrix.

It is the object of the present invention to provide a method for producing a separating material, whereby the above-mentioned disadvantages of the prior art are overcome.

Accordingly, the present invention provides a method for the production of a separating material by:
a) providing a solid substrate, having amino-functional groups coupled to the substrate surface,
b) covalently coupling of the amino-functional groups with a thermally labile radical initiator,
c) contacting the substrate surface with a solution of polymerizable monomers at a temperature of from 50°C to 120°C, wherein the thermally initiated graft copolymerisation of the monomers is carried out in aqueous solution.

One advantage of the present invention is that the method of producing the separating material of the present invention does not require an organic solvent, such as carbon tetrachloride, which is difficult to remove from the final product, and which may be toxic or at least harmful to a patient, when the separating material is used in medical applications and extra-corporeally contacted to any liquid or body fluid, which is (re-)introduced into the patient's body.

Another advantage of the present invention lies in the covalently coupling of the radical initiator to the aminofunctional groups on the solid substrate. Thereby, the occurrence of homopolymerization in the reaction solution is avoided or at least minimized. The radical initiator, which is bound to the solid substrate, forms radicals upon temperature increase, and part of the radical initiator structure becomes part of the polymer chains, which are formed from the solid substrate surface. The polymer chains of the present invention develop from the surface of the substrate without the formation of undesired cross-linkages between the chains, thus the process of the present invention is considered to provide a very "clean" chemistry.

Another advantage of the present invention is based on the use of thermally labile radical initiators, which can be chosen to ensure mild reaction conditions and to avoid additional reactants which may react with the substrate or the monomers in an undesired manner. The temperatures to initiate radical formation of useful radical initiators lie within the range of 50°C to 120°C, preferably in the range of 70°C to 100°C. A useful temperature range of the polymerization reaction is from the 10 hour half life temperature of the radical initiator to 20 to 25 degrees above that 10 hour half life temperature. By adjusting the reaction temperature it is further possible to very precisely control the polymerization reaction, e. g. onset of the reaction, reaction speed, degree of polymerization.

In a preferred embodiment of the present invention, the solid substrate is a porous polymeric material. The porosity of the substrate material provides a large surface area for the contact between the separating material and the fluid.

An advantageous use of the separating material is the medical application in the extracorporeal treatment of human or animal blood or other body fluids, e. g. hemodialysis, filtration, and/or removal of undesired substances from the blood by adsorption of such substances to the separating material. Usually, in such applications the blood of a patient is extracorporeally separated into the blood cells and the blood plasma (or blood serum), the latter containing most of the substances to be removed by the treatment. In another preferred embodiment of the invention the porous polymeric material has a pore size that is sufficiently large to allow passage of blood plasma, or blood serum through the substrate material. This allows the blood plasma or blood serum to get in contact with the entire surface area within the pores of the separating material. In another embodiment the porous polymeric material has a pore size that is sufficiently large to allow passage of blood plasma, or blood serum through the substrate material, whereby the pore size is sufficiently small to avoid passage of the blood cells. This allows the use of the separation material, if it is in the form of a membrane or a hollow fibre membrane, to separate the blood cells from the blood plasma by passing whole blood onto or by the membrane. Thereby, the blood cells are retained on one side of the membrane, whereas blood plasma can pass through the pores of the membrane to the opposite side of the membrane. Thus, blood cells are filtered from the blood plasma. While the blood plasma is passing the separating material membrane, it is contacted within the pores of the material to the active surface of the material. Thereby, the separating material depletes the blood plasma from undesired substances by adsorption. Afterwards, the depleted or purified blood plasma may be recombined with the blood cells and for example be reinjected into the patient's circulation, or it may be stored for later use.

The separating material of the present invention may be provided in any form, but preferably is in the form of a membrane, a hollow fibre membrane, a particle bed, a fibre mat, or beads. Most preferred it is in the form of a hollow fibre membrane, as it is for example well known from hemodialysis applications. Multiple hollow fibre membranes can by known procedures be potted into tubes, and the tubes being fitted with ports in a known manner, to provide separating units, which preferably are in the form of cartridges useful to be inserted into dialysis apparatuses. If the separating material of the present invention is provided in the form of beads, such beads can for example be packed into columns for the passage of the fluid to be treated, e. g. blood plasma.

In another preferred embodiment the separating material is made of a biocompatible material, to avoid any hazardous effects on a treated body liquid of a patient or on the patient itself, if the treated liquid is reinfused into the patient.

Preferred materials useful for the preparation of the separating material of the present invention are selected from the group, consisting of polyacrylates, polystyrene, polyethylene oxide, cellulose, cellulose derivatives, polyethersulfone (PES), polypropylene (PP), polysulfone (PSU), polymethylmethacrylate (PMMA), polycarbonate (PC), polyacrylonitrile (PAN), polyamide (PA), polytetrafluorethylene (PTFE), cellulose acetate (CA), regenerated cellulose, and blends or copolymers of the foregoing, or blends or copolymers with hydrophilizing polymers, preferably with polyvinylpyrollidone (PVP) or polyethyleneoxide (PEO).

Preferably, the amino-functional groups on the solid substrate for the production of the separating material of the invention are primary amino groups, even though secondary amino groups may also be useful. Primary amino groups provide for a higher reactivity.

In a highly preferred embodiment of the present invention, the thermally labile radical initiator, as the starting material before coupling to the amine groups on the solid substrate, comprises at least one, preferably two carboxylic groups. In the reaction of coupling of the radical initiator to the amine group of the substrate, the carboxylic groups are preferably activated by a water soluble carbodiimide, for example 1-ethyl-3-(3-dimethylaminopropyl)-carbodiimide (EDAC) which forms active o-acylurea intermediates. After initial activation, the carboxyl groups will react with e.g. N-hydroxysuccinimide (NHS) to form an active ester, which couples with the primary amino groups on the surface of the substrate.

Useful thermally labile radical initiators include compounds which decompose to give free radicals on thermal activation. Preferably, the thermally labile radical initiator being selected among azo compounds or peroxides. Most preferred radical initiators are 4,4'-azobis-(4-cyanovaleric acid) or 2,2'-azobis-[N-(2-carboxyethyl)-2-methylpropionamidine.

The monomers useful to form the polymer chains from the substrate surface by graft polymerization are selected from compounds having a polymerizable double bond. Preferred monomers can be divided into three groups: (1) monomers providing positive or negative charges, (2) monomers for binding affinity ligands, and (3) inert hemocompatible monomers.

Preferred monomers of the first group (1) are selected from N,N-Dimethylaminoethyl acrylamide, N,N-Diethylaminoethyl acrylamide, N,N-Dimethylaminopropyl actylamide (DMPA), N,N-Dimethylaminopropyl methactylamide, N,N-Dimethylaminoethyl methacrylate, N,N-Dieethylaminoethyl methacrylate, N,N-Dimethylaminoethyl actylate, N-Morpholinoethylactylate, N-Morpholinoethyl methacrylate, 1-Vinylimidazole, Trimethylammoniumethyl acrylamide, Trimethylammoniumpropyl methacrylamide, and Trimethylammoniumethyl methacrylate. The most preferred monomer of this group is Dimethylaminopropyl actylamide (DMPA).

Preferred monomers of the second group (2) are selected from Glycidyl acrylate, Glycidyl methactylate, Vinylglycidyl ether, and Vinyl glycidyl urethane. The most preferred monomer of this group is Glycidyl methacrylate.

Preferred monomers of the third group (3) are selected from 2-Hydroxyethyl methacrylate, 2-Hydroxypropyl methactylate, Hydroxymethyl methacrylate, N-Vinylpyrrolidone, 2-Vinyl pyridine, 4-Vinyl pyridine, and N-Vinyl-2-methylimidazole. The most preferred monomer of this group is 2-Hydroxyethyl methacrylate.

The polymerization reaction can comprise one single type of monomer of the above-mentioned, or it can be carried out using two or more different types of monomers of the same or different of the above groups.

Highly preferred polymerizable monomers are selected from compounds of the following formula:

H₂C=C(R¹)-C (O)-X-R²-N(R³)₂,

wherein R¹= hydrogen, methyl or ethyl group; R²= C1-C6-alkyl or aryl group; R³= methyl or ethyl group; and X=NH or 0.

The invention will now be described and further illustrated by way of preferred reaction schemes, examples and the accompanying figures.
Figure 1 illustrates data of the measurement of the endotoxin concentrations according to example 9, described below.
Figure 2 illustrates the experimental set-up for dynamic endotoxin adsorption of grafted membranes from citrate-coagulated human blood according to example 10, described below.
Figure 3 illustrates data of the measurement of the endotoxin concentrations in filtrates according to example 10, described below.
Figure 4 illustrates data of the measurement of the endotoxin concentrations in the blood reservoir according to example 10, described below.

### Preferred Reaction Schemes

1. For illustration purposes, by way of example the separating material of the present invention can be produced, e, g, using N,N-dimethylaminopropylacrylamide as the polymerizable monomer. This monomer provides a basic group which is positively charged at physiologic pH. Therefore, the produced separating material is effective to adsorb negatively charged substances by charge interaction, e. g. bacterial toxins such as endotoxins from gram-negative bacteria, lipoteichoic acid from gram-positive bacteria or bacterial DNA. Using the produced separating material, a number of tests have been carried out with respect to endotoxin removal from plasma or blood (see below). The EP 1 518 870 A1 production of the thus produced separating material is illustrated in reaction scheme 1 below.

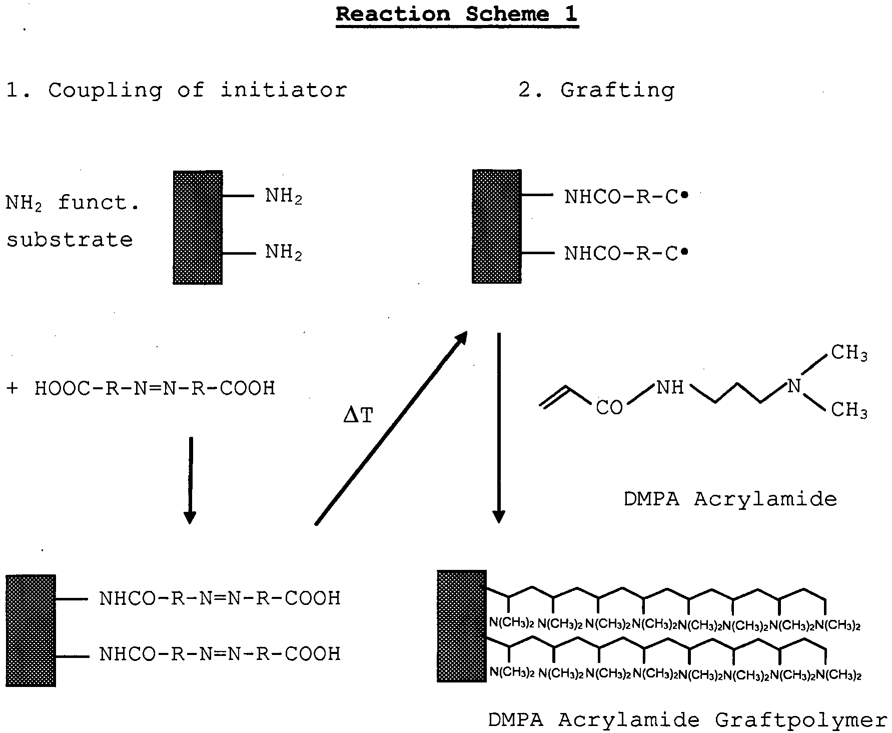

In the first reaction step the polymerisation initiator is covlently coupled to the support. Therefore, the amino-group containing supports are reacted with activated esters, e.g. carbodiimide or anhydride activated carboxylic groups of the initiator. Thereby the polymerization initiator is bound to the activated sites. Suitable polymerization initiators are compounds which decompose to give free radicals at thermal activation, e.g. azo compounds or peroxides, and which further carry reactive substituents, e.g. carboxylic groups. Particularly preferred initiators are azo carboxyl compounds, such as 4,4'-azobis(4-cyanovaleric acid) or 2,2'-azobis[N-(2-carboxyethyl)-2-methylpropionamidine] The carboxyl groups are preferably activated by the water soluble carbodiimide 1-ethyl-3-(3-dimethylaminopropyl)-carbodiimide (EDAC) which forms active O-acylurea intermediates. After initial activation by EDAC, the carboxyl groups will react with N-hydroxysuccinimide (NHS) to form an active ester, which couples with the primary amino groups on the surface of the substrate. The reaction can be carried out in aqueous solution at a pH > 12, which is preferable for medical applications.

Concentration ranges of compounds for modification of 1-10 g substrate (membrane or beads): 20 - 200 ml solvent
0.1-10 g 4,4'-Azobis(4-cyanovaleric acid)
0.2-20 g EDA
0.2-20g NHS

| | |
|---|---|
| Reaction temperature: | **0-30** °C, most preferably room temperature for simplicity reasons |
| Reaction time: | 1 to a several hours |

In the second reaction step the initiator immobilized surface is contacted with a solution of N,N-dimethylaminopropyl acrylamide. The reaction can be carried out in degassed water in an inert atmosphere. The temperature is chosen above the 10 hour half life temperature of the initiator. The grafting reaction typically uses 1 to 20 g N,N-dimethylaminopropyl acrylamide dissolved in from 10 to 300 ml water. The reaction is typically carried out at from 70 to 95°C, and typically takes from 30 min to several hours.

Other monomers suitable for introducing positive charges are, inter alia, N,N-dimethylaminoethyl acrylamide, N,N-diethylaminoethyl acrylamide, N,N-dimethylaminoethyl methacrylate N,N-dimethylaminoethyl acrylamide, N,N-dimethylaminopropyl methacrylamide and trimethylammoniumethyl acrylamide.

2. In another illustrating example the separating material of the present invention can be produced, e, g, using glycidyl methacrylate as the polymerizable monomer. This type of monomer is used for subsequent binding of affinity ligands such as proteins, peptides, antibodies or other biological molecules. The epoxide groups can also be reacted with amino compounds, such as diethylamine, triethylamine or arginine, to give positively charged adsorbents. Other monomers which comprise both a polymerizable double bond and an oxirane ring are for example glycidyl acrylate, vinyl glycidyl ether and vinyl glycidyl urethane. The production of the thus produced separating material is illustrated in reaction scheme 2 below.

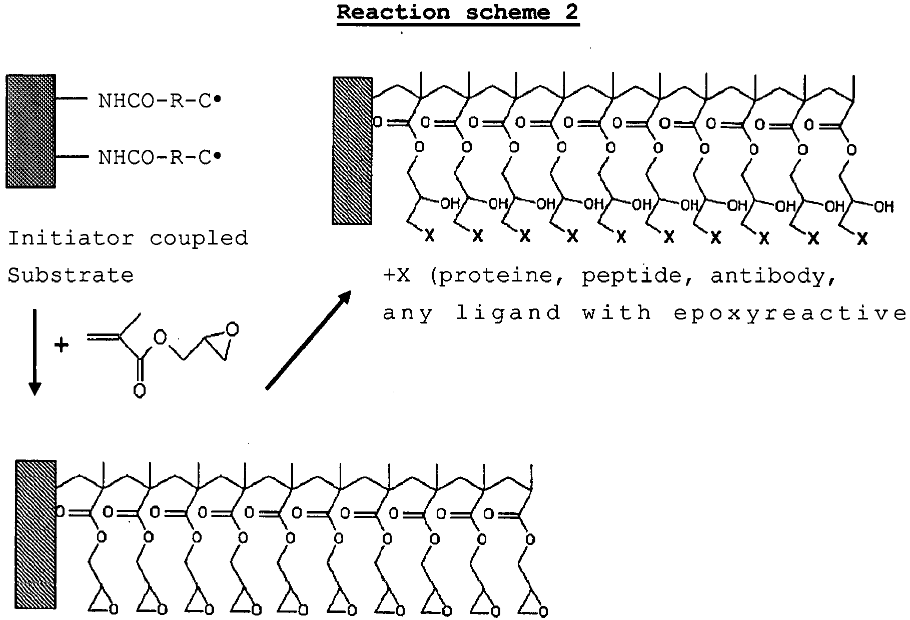

In the first reaction step coupling of polymerisation initiator is performed as described above in connection with reaction scheme 1. In the second reaction step the surface is contacted again with a solution of the monomer at elevated temperature in an inert atmosphere.

Reaction components:
1-10 g Substrate (membrane or beads)
1-20 g Glycidyl methacrylate
10-400 ml solvents (water, ethanol, ethanol, toluene, DMF, DMSO)

3. In another illustrating example the separating material of the present invention can be produced using a mixture of a functional monomer and an inert monomer as the polymerizable monomers. The monomers can be used alone, as also illustrated above, or in a mixture with inert monomers, e.g. vinyl pyrrolidone, hydroxymethyl methacrylamide or hydroxyethyl actylate in order to increase the hydrophilicity of the polymer or/and to improve the biocompatibility of the materials. The production of the thus produced separating material is illustrated in reaction scheme 3 below.

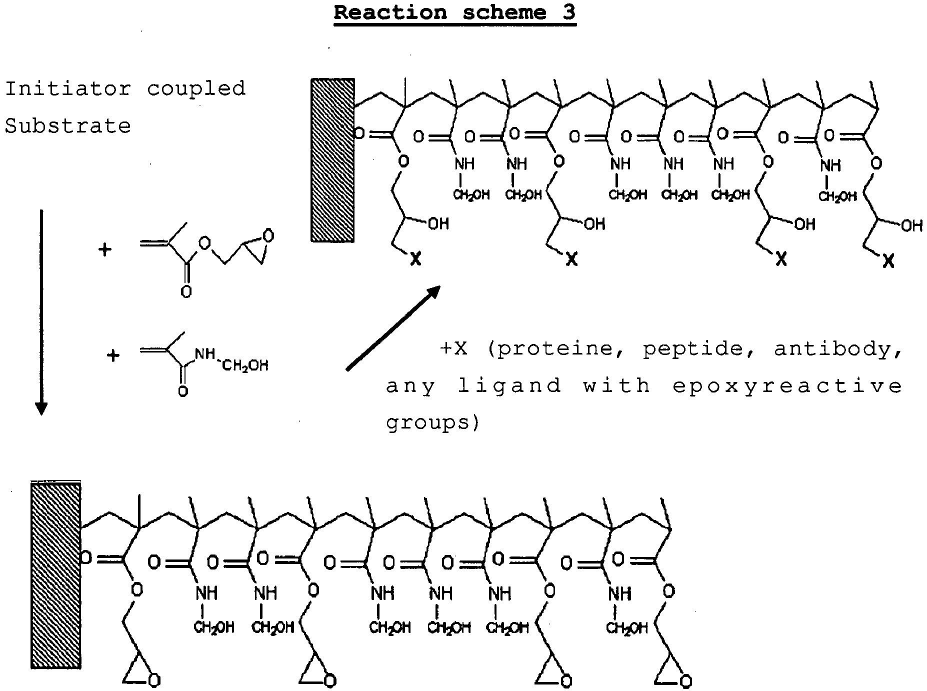

### Example 1 Coupling of 4,4'-azobis(4-cyanovaleric acid) onto macroporous acrylic beads

200 g oxirane acrylic resin beads (e.g. Toyo Pearl HW70EC, Tosoh Corp.) having an average epoxy group content of 4.0 mmollg are aminated with 150 ml conc. ammonia solution (32 wt%) for 3 hours at 40 "C. After washing with distilled water, 45 g beads are resuspended in 400 ml DMF and 10 g 4,4'-azobis(4-cyanovaleric acid). 15 g EDAC and 15 g NHS are added. The batch is agitated for 12 hours at room temperature and afterwards rinsed with water.

### Example 2 Coupling of 4,4'-azobis(4-cyanovaleric acid) onto microporous hollow fiber membranes

A bundle of polyethersulfon/polyvinylpyrrolidone hollow fibre membranes (144 fibres, 25 cm long, inner diameter 260 pm, outer diameter 340 pm, mean pore diameter 0.3 pm, functionalised with 1.5 pmol/g primary amino groups by plasma treatment as described in Swedish patent application 020107-8) is incubated with 0.6 g 4,4'-azobis (4-cyanovaleric acid) and 0.85 g NHS in 45 ml 0.1 M NaOH. Then 0.85 g EDAC dissolved in 5 ml 0.1 M NaOH is added and agitated for 12 h at room temperature. Afterwards the excess reagents are removed by washing repeatedly with water.

### Example 3 Graft polymerisation of beads with N,N-dimethylaminopropylacrylamide

15 g beads derivatized as in Example 1 are reacted in a reaction solution of 0.45 g N,N-dimethylaminopropylacrylamide in 75 ml degassed water in a three-necked flask. The reaction is performed with gentle stirring at 75°C for 3 hours in an atmosphere of nitrogen. The derivatized beads are then rinsed as follows: 1 I hot water, 50 ml 1 M NaOH, 50 ml 1 M HCl, 0.5 l water, 0.5 l PBS buffer (pH=7.4), 0.5 l 1 M NaCl solution and 0.5 1 water.

### Example 4 Graft polymerisation of beads with glycidyl methacrylate

20 g beads derivatized as in Example 1 are reacted in a reaction solution of 8.0 g glycidyl methacrylate in 120 ml toluene in a three-necked flask. The reaction is performed with gentle stirring at 75°C for 3 hours in an atmosphere of nitrogen (reflux condenser). The derivatized beads are then thoroughly rinsed as described in example 3 and dried overnight at 40°C in a vacuum drying oven. The degree of grafting is found to be 125 %.

### Example 5 Graft polymerisation of microporous membranes with N,N-dimethylaminopropyl actylamide

A bundle of membranes derivatized as in Example 2 is reacted in a reaction solution of 2,5 g N,N-dimethylaminopropyl acrylamide in 40 ml degassed water in a three-necked flask. The reaction is performed with gentle stirring at 75°C for 12 hours in an atmosphere of nitrogen. The derivatized membranes are then thoroughly rinsed as described in example 3. To prepare a membrane device the bundle was dried and potted at each end of a 10 mm-diameter poly (carbonate) tube fitted with two ports in the shell.

### Example 6 Graft polymerisation of microporous membranes with glycidyl methacrylate

A bundle of membranes derivatized as in Example 2 is reacted in a reaction solution of 1.0 g glycidyl methacrylate in 40 ml Isopropanol water in a three-necked flask. The reaction is performed with gentle stirring at 75°C for 3 hours in an atmosphere of nitrogen. The derivatized membranes are then thoroughly rinsed as described in example 3 and reacted with 2.0 g oligo arginine in 40 g water, which has been synthesized as described in W00123413.

### Example 7 Graft polymerisation of microporous membranes with a mixture of glycidyl methacrylate and hydroxymethyl methactylamide

A bundle of membranes derivatized as in Example 2 is reacted in a reaction solution of 0.6 g glycidyl methacrylate and 2.4 g hydroxymethyl methacrylamide in 40 ml degassed water in a three-necked flask. The reaction is performed with gentle stirring at 75°C for 3 hours in an atmosphere of nitrogen. The derivatized membranes are then thoroughly rinsed as described in example 3 and dried overnight at 40°C in a vacuum drying oven. The degree of grafting is found to be 122.5 %.

### Example 8 Determination of the grafting yield and protein binding capacity

The dynamic protein binding capacity of the adsorbents produced in examples 3, 5, and 6 was determined by establishing the breakthrough curves of the membrane modules or fixed bed columns filled with the grafted beads in dead-end filtration mode and single-pass perfusion mode, respectively. Thereby a solution of bovine serum albumin (1 g/l in 20 mM Tris pH 8.0) is pumped through the modules or columns at a perfusion rate of 1 ml/min. The effluent is monitored by a flow-through UV-detector cell at 280 nm. The results are shown in the following table 1.

**Table 1**

| Adsorbent | Degree of grafting [%] | Dynamic BSA binding capacity [mg/g substrate] |
|---|---|---|
| Example 3 | 117 | 180 |
| Example 5 | 104 | 166 |
| Example 6 | 108 | 99 |

### Example 9 Dynamic endotoxin adsorption of grafted beads from citrate-anticoagulated human blood

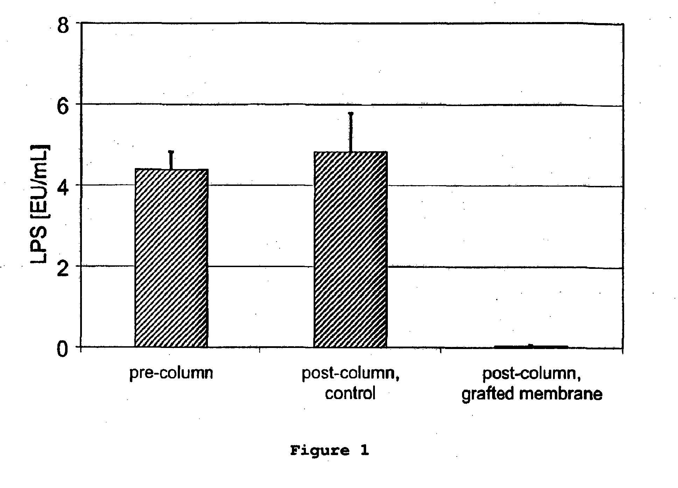

4 g beads grafted with N,N-dimethylaminopropylactylamide, as described in example 3, are packed into a poly(carbonate) column. A column with 4 g acrylate beads not reacted with any ligand is used as control. To eliminate potential contamination the column is perfused with 100 ml 30% ethanol (0.1 M NaOH, 8.8 gll NaCI), followed by 200 ml RingerIACD solution and 100 ml pyrogen-free 0.9% saline. Endotoxin (LPS from E. coli, 055B.5) is added to freshly donated citrate anticoagulated human blood at a concentration of 10 EU/ml. 150 ml blood prepared in this manner is then passed through the columns at a flow rate of 1.3 ml/min. Aliquots of 1 ml were taken before and after the test columns and assayed for LPS content using chromogenic Limulus Amebocyte Lysate (LAL) test (Charles River Endosafe, Inc.) as described by K. Duner, (1993) Journal of Biochem. and Biophys. Method 26:131-142. The results are shown in Fig. 1.

### Example 10 Dynamic Endotoxin adsorption of grafted membranes from citrate-anticoagulated human blood

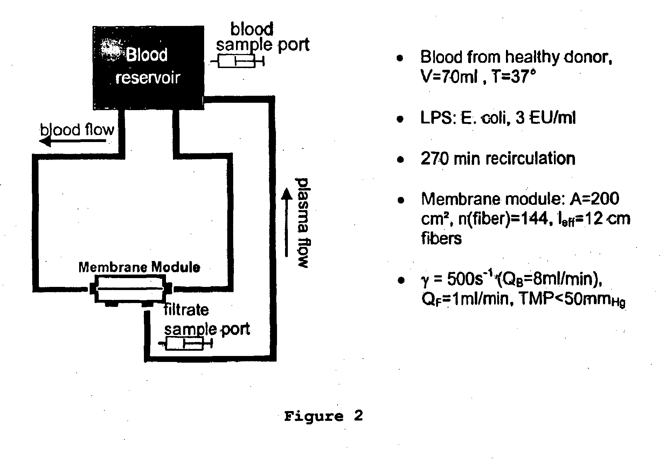

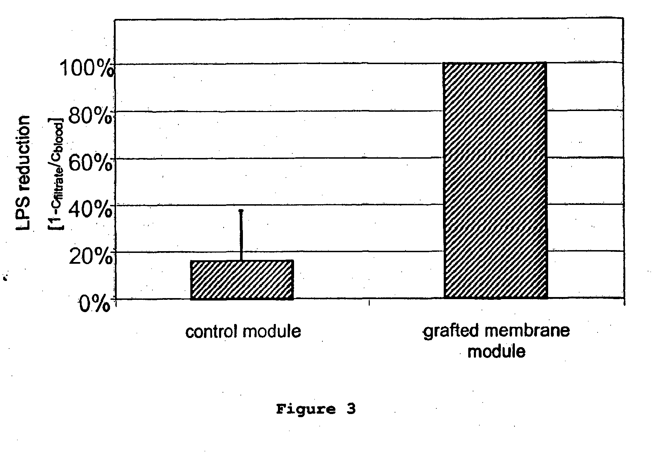

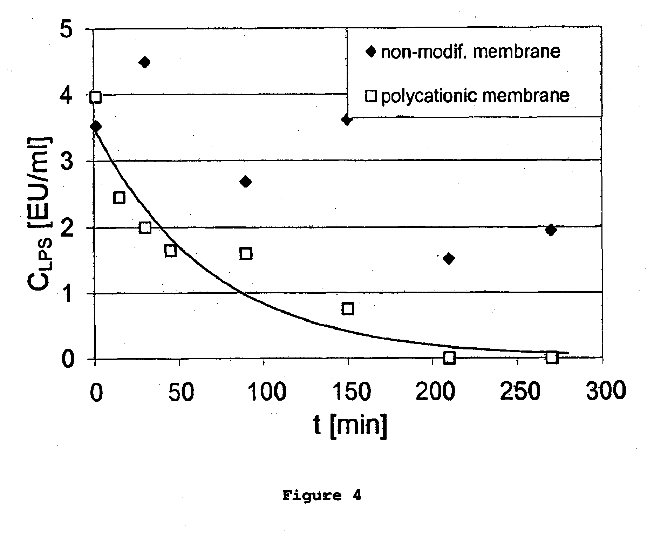

Membrane modules prepared as in Example 5 were sterilized with steam and rinsed in filtration mode with 200 ml pyrogen-free 0.9% saline. Then 90 ml Citrate-anticoagulatede fresh human whole blood spiked with 3 EU/ml Endotoxin (LPS from E. coli, 055B.5) was perfused from a blood reservoir through the module under recirculating conditions, as it is shown in figure 2. The blood flow rate was 8 ml/min and plasma is filtrated at a flow rate of 1 ml/min through the membrane of the membrane module. To avoid a dilution effect the first 20 ml blood were withdrawn after perfusion. After 30 min, 90 min, 150 min, 210 min, and 270 min aliquots of 1 ml were taken from the filtrate and from the blood reservoir, as illustrated in figure 2 (filtrate sample port; blood sample port), and assayed for LPS content using the LAL test described in example 9. A membrane module with membranes not modified with ligands is used in a control experiment. As shown in figure 3 the spiked endotoxin was completely removed from the plasma fraction filtrated through the membrane. As shown in figure 4, the treatment resulted in a 100 % reduction of endotoxin in the blood pool after 210 minutes of perfusion.

## Claims

1. A method for the production of a separating material by:
a) providing a solid substrate, having amino-functional groups coupled to the substrate surface,
b) covalently coupling of the amino-functional groups with a thermally labile radical initiator, and
c) contacting the substrate surface with a solution of polymerizable monomers at a temperature of from 50°C to 120°C, wherein the thermally initiated graft copolymerization of the monomers is carried out in aqueous solution.

2. The method of claim 1, wherein the solid substrate is a porous polymeric material with a pore size to allow passage of blood, blood plasma, or blood serum through the substrate material.

3. The method of any of claims 1 and 2, wherein the solid substrate is in the form of a membrane, a hollow fiber membrane, a particle bed, a fiber mat, or beads.

4. The method of any of claims 1 to 3, wherein the solid substrate is made of a biocompatible material.

5. The method of any of claims 1 to 4, wherein the solid substrate is made of a material selected from the group, consisting of polyacrylates, polystyrene, polyethylene oxide, cellulose, cellulose derivatives, polyethersulfone (PES), polypropylene (PP), polysulfone (PSU), polymethylmethacrylate (PMMA), polycarbonate (PC), polyacrylonitrile (PAN), polyamide (PA), polytetrafluorethylene (PTFE), cellulose acetate (CA), regenerated cellulose, and blends or copolymers of the foregoing, or blends or copolymers with hydrophilizing polymers, preferably with polyvinylpyrollidone (PVP) or polyethyleneoxide (PEO).

6. The method of any of claims 1 to 5, wherein the amino-functional groups are primary amino groups.

7. The method of any of claims 1 to 6, wherein the thermally labile radical initiator comprises at least one carboxylic group.

8. The method of any of claims 1 to 7, wherein the thermally labile radical initiator includes compounds which decompose to give free radicals on thermal activation, preferably the thermally labile radical initiator being selected among azo-compounds or peroxides.

9. The method of any of claims 1 to 8, wherein the thermally labile radical initiator is 4,4'-azobis-(4-cyanovaleric acid) or 2,2'-azobis-[N-(2-carboxyethyl)-2-methylpropionamide.

10. The method of any of claims 1 to 9, wherein the polymerizable monomers are selected from compounds having a polymerizable double bond.

11. The method of any of claims 1 to 10, wherein the polymerizable monomers are selected from the group consisting of acrylic acid, methacrylic acid, vinyl compounds, and derivatives of the foregoing compounds, N,N-dimethylaminoethyl acrylamide, N,N-diethylaminoethyl acrylamide, N,N-dimethylaminopropyl acrylamide (DMPA), N,N-dimethylaminopropyl methacrylamide, N,N-dimethylaminoethyl methacrylate, N,N-diethylaminoethyl methacrylate, N,N-dimethylaminoethyl acrylate, N-morpholinoethyl acrylate, N-morpholinoethyl methacrylate, 1-vinylimidazole, trimethylammoniumethyl acrylamide, trimethylammoniumpropyl methacrylamide, trimethylammoniumethyl methacrylate, glycidyl acrylate, glycidyl methacrylate, vinyl glycidyl ether, vinyl glycidyl urethane, 2-hydroxyethyl methacrylate, 2-hydroxypropyl methacrylate, hydroxymethyl methacrylate, N-vinylpyrrolidone, 2-vinyl pyridine, 4-vinyl pyridine, N-vinyl-2-methylimidazole.

12. The method of any of claims 1 to 11, wherein the polymerizable monomers comprise dimethylaminopropyl acrylamide (DMPA).

13. The method of any of claims 1 to 12, wherein the polymerizable monomers are selected from compounds of the following formula:
H₂C=C(R¹)-C(O)-X-R²-N(R³)₂,
wherein R¹= hydrogen, methyl or ethyl group; R²= alkyl or aryl group; R³= methyl or ethyl group; and X=NH or 0.

## Patentansprüche

1. Ein Verfahren zur Herstellung eines Trennmaterials, indem man
(a) ein festes Substrat vorlegt, an dessen Substratoberfläche sich funktionelle Aminogruppen befinden,
(b) die funktionellen Aminogruppen kovalent mit einem thermolabilen Radikalinitiator verknüpft, und
(c) die Substratoberfläche bei einer Temperatur von 50°C bis 120°C mit einer Lösung eines polymerisierbaren Monomers in Kontakt bringt, wobei die durch Wärme initiierte Pfropfpolymerisation der Monomere in wässriger Lösung durchgeführt wird.

2. Das Verfahren gemäß Anspruch 1, wobei das feste Substrat ein poröses polymeres Material mit einer Porengröße ist, die den Durchtritt von Blut, Blutplasma oder Blutserum durch das Substratmaterial gestattet.

3. Das Verfahren gemäß einem der Ansprüche 1 und 2, wobei das feste Substrat in Form einer Membran, einer Hohlfasermembran, eines Partikelbetts, einer Fasermatte oder von Beads vorliegt.

4. Das Verfahren gemäß einem der Ansprüche 1 bis 3, wobei das feste Substrat aus biokompatiblem Material besteht.

5. Das Verfahren gemäß einem der Ansprüche 1 bis 4, wobei das feste Substrat aus einem Material besteht, welches ausgewählt wird aus der Gruppe bestehend aus Polyacrylaten, Polystyrol, Polyethylenoxid, Cellulose, Cellulosederivaten, Polyethersulfon (PES), Polypropylen (PP), Polysulfon (PSU), Polymethylmethacrylat (PMMA), Polycarbonat (PC), Polyacrylnitril (PAN), Polyamid (PA), Polytetrafluorethylen (PTFE), Celluloseacetat (CA), regenerierte Cellulose und Mischungen oder Copolymere der vorstehend genannten Materialien, oder Mischungen oder Copolymere mit hydrophilen Polymeren, vorzugsweise mit Polyvinylpyrrolidon (PVP) oder Polyethylenoxid (PEO).

6. Das Verfahren gemäß einem der Ansprüche 1 bis 5, wobei die funktionellen Aminogruppen primäre Aminogruppen sind.

7. Das Verfahren gemäß einem der Ansprüche 1 bis 6, wobei der thermolabile Radikalinitiator zumindest eine Carboxylgruppe enthält.

8. Das Verfahren gemäß einem der Ansprüche 1 bis 7, wobei der thermolabile Radikalinitiator Verbindungen umfasst, die bei der Zersetzung durch thermische Aktivierung Radikale freisetzen, und wobei der thermolabile Radikalinitiator vorzugsweise aus Azoverbindungen oder Peroxiden ausgewählt wird.

9. Das Verfahren gemäß einem der Ansprüche 1 bis 8, wobei der thermolabile Radikalinitiator 4,4'-Azobis (4-cyanovaleriansäure) oder 2,2'-Azobis[N-(2-carboxyethyl)-2-methylpropionamidin ist.

10. Das Verfahren gemäß einem der Ansprüche 1 bis 9, wobei die polymerisierbaren Monomere aus Verbindungen ausgewählt werden, die eine polymerisierbare Doppelbindung besitzen.

11. Das Verfahren gemäß einem der Ansprüche 1 bis 10, wobei die polymerisierbaren Polymere ausgewählt werden aus der Gruppe bestehend aus Acrylsäure, Methacrylsäure, Vinylverbindungen und Derivaten der vorstehend genannten Verbindungen, N,N-Dimethylaminoethyl acrylamid, N,N-Diethylaminoethyl acrylamid, N, N-Dimethylaminopropyl acrylamid (DMPA), N, N-Diemethylaminopropyl methacrylamid, N,N-Dimethylaminoethyl methacrylat, N,N-Diethylaminoethyl methacrylat, N,N-Dimethylaminoethyl acrylat, N-Morpholinoethyl acrylat, N-Morpholinoethyl methacrylat, 1-Vinylimidazol, Trimethylammoniumethyl acrylamid, Trimethylammoniumpropyl methacrylamid, Trimethylammoniumethyl methacrylat, Glycidylacrylat, Glycidylmethacrylat, Vinylglycidylether, Vinylglycidylurethan, 2-Hydroxyethyl methacrylat, 2-Hydroxypropyl methacrylat, Hydroxymethyl methacrylat, N-Vinylpyrrolidon, 2-Vinylpyridin, 4-Vinylpyridin, N-Vinyl-2-methylimidazol.

12. Das Verfahren gemäß einem der Ansprüche 1 bis 11, wobei die polymerisierbaren Monomere Dimethylaminopropyl acrylamid (DMPA) umfassen.

13. Das Verfahren gemäß einem der Ansprüche 1 bis 12, wobei die polymerisierbaren Monomere aus Verbindungen der folgenden Formel ausgewählt werden:
H₂C=C(R¹)-C(O)-X-R²-N(R³)₂,
wobei R¹ = Wasserstoff, Methyl oder Ethyl; R² = Alkyl- oder Aryl; R³ = Methyl- oder Ethyl; und X = NH oder O.

## Revendications

1. Procédé de production d'un matériau de séparation par :
a) fourniture d'un substrat solide, ayant des groupes amino-fonctionnels couplés à la surface du substrat,
b) couplage covalent des groupes amino-fonctionnels à un initiateur de radicaux thermiquement labiles, et
c) mise en contact de la surface du substrat avec une solution de monomères polymérisables à une température de 50 °C à 120 °C, dans lequel la copolymérisation par greffage initiée thermiquement des monomères est réalisée dans une solution aqueuse.

2. Procédé selon la revendication 1, dans lequel le substrat solide est un matériau polymère poreux ayant une taille de pores permettant le passage du sang, du plasma sanguin ou du sérum sanguin à travers le matériau de substrat.

3. Procédé selon l'une quelconque des revendications 1 et 2, dans lequel le substrat solide est sous la forme d'une membrane, d'une membrane à fibres creuses, d'un lit de particules, d'une nappe de fibres, ou de billes.

4. Procédé selon l'une quelconque des revendications 1 à 3, dans lequel le substrat solide est fait d'un matériau biocompatible.

5. Procédé selon l'une quelconque des revendications 1 à 4, dans lequel le substrat solide est fait d'un matériau choisi dans le groupe constitué des polyacrylates, du polystyrène, du poly(oxyde d'éthylène), de la cellulose, des dérivés de cellulose, de la polyéthersulfone (PES), du polypropylène (PP), de la polysulfone (PSU), du poly(méthacrylate de méthyle) (PMMA), du polycarbonate (PC), du polyacrylonitrile (PAN), du polyamide (PA), du polytétrafluoroéthylène (PTFE), de l'acétate de cellulose (CA), de cellulose régénérée, et de mélanges ou de copolymères de ce qui précède ou de mélanges ou de copolymères avec des polymères d'hydrophilisation, de préférence avec la polyvinylpyrrolidone (PVP) ou le poly(oxyde d'éthylène) (PEO).

6. Procédé selon l'une quelconque des revendications 1 à 5, dans lequel les groupes amino-fonctionnels sont des groupes amino primaires.

7. Procédé selon l'une quelconque des revendications 1 à 6, dans lequel l'initiateur de radicaux thermiquement labiles comprend au moins un groupe carboxylique.

8. Procédé selon l'une quelconque des revendications 1 à 7, dans lequel l'initiateur de radicaux thermiquement labiles comprend des composés qui se décomposent pour donner des radicaux libres suite à une activation thermique, de préférence l'initiateur de radicaux thermiquement labiles est choisi parmi les composés azoïques ou les peroxydes.

9. Procédé selon l'une quelconque des revendications 1 à 8, dans lequel l'initiateur de radicaux thermiquement labiles est le 4,4'-azobis-(acide 4-cyanovalérique) ou le 2,2'-azobis-[N-(2-carboxyéthyl)-2-méthylpropionamide.

10. Procédé selon l'une quelconque des revendications 1 à 9, dans lequel les monomères polymérisables sont choisis parmi les composés comprenant une double liaison polymérisable.

11. Procédé selon l'une quelconque des revendications 1 à 10, dans lequel les monomères polymérisables sont choisis dans le groupe constitué de l'acide acrylique, de l'acide méthacrylique, des composés vinyliques et des dérivés des composés précédents, du N,N-diméthylaminoéthylacrylamide, du N,N-diéthylaminoéthylacrylamide, du N,N-diméthylaminopropylacrylamide (DMPA), du N,N-diméthylaminopropylméthacrylamide, du méthacrylate de N,N-diméthylaminoéthyle, du méthacrylate de N,N-diéthylaminoéthyle, de l'acrylate de N,N-diméthylaminoéthyle, de l'acrylate de N-morpholinoéthyle, du méthacrylate de N-morpholinoéthyle, du 1-vinylimidazole, du triméthylammoniuméthylacrylamide, du triméthylammoniumpropylméthacrylamide, du méthacrylate de triméthylammoniuméthyle, de l'acrylate de glycidyle, du méthacrylate de glycidyle, du vinylglycidyléther, du vinylglycidyluréthane, du méthacrylate de 2-hydroxyéthyle, du méthacrylate de 2-hydroxypropyle, du méthacrylate d'hydroxyméthyle, de la N-vinylpyrrolidone, de la 2-vinylpyridine, de la 4-vinylpyridine, du N-vinyl-2-méthylimidazole.

12. Procédé selon l'une quelconque des revendications 1 à 11, dans lequel les monomères polymérisables comprennent du diméthylaminopropylacrylamide (DMPA).

13. Procédé selon l'une quelconque des revendications 1 à 12, dans lequel les monomères polymérisables sont choisis parmi les composés de formule suivante :
H₂C=C(R')-C(O)-X-R²-N(R³)₂,
dans laquelle R¹ = hydrogène, un groupe méthyle ou éthyle ; R² = un groupe alkyle ou aryle ; R³ = un groupe méthyle ou éthyle ; et X = NH ou O.
